# EUROPEAN PATENT SPECIFICATION

(11) **EP 1 278 823 B1**
(45) Date of publication and mention of the grant of the patent: **27.07.2011**
(21) Application number: 01931242.0
(22) Date of filing: 01.05.2001
(51) Int. Cl.: C12N 5/07, A01N 1/02, A61L 2/00, A61K 35/12, A61K 39/42

(54) **CLEARANCE OF NEOPLASTIC CELLS FROM MIXED CELLULAR COMPOSITIONS USING VIRUSES**
ENTFERNUNG VON NEOPLASTISCHEN ZELLEN AUS GEMISCHTEN ZELLULÄREN ZUSAMMENSETZUNGEN MITTELS VIREN
CLAIRANCE VIRALE DE CELLULES NEOPLASTIQUES DE COMPOSITIONS CELLULAIRES MIXTES

(30) Priority: 03.05.2000 US 201990 P; 19.05.2000 US 205389 P; 13.02.2001 US 268054 P; 16.03.2001 US 276782 P
(43) Date of publication of application: 29.01.2003
(60) Divisional application: 10181628.8
(73) Proprietor: Oncolytics Biotech Inc., Calgary, AB T2N 1X7 (CA)
(72) Inventor: MORRIS, Donald, Calgary, Alberta T2Z 2S1 (CA); THOMPSON, Bradley, G., Calgary, Alberta T2N 0Z5 (CA); COFFEY, Matthew, C., Calgary, Alberta T2N 3L4 (CA)
(74) Representative: Nash, David Allan
(86) International application number: PCT/CA2001/000609
(87) International publication number: WO 2001/083710

(56) References cited:
- WO-A2-2005/007824
- US-A- 2004 109 878
- US-A1- 2004 109 878
- SETH P ET AL: "Adenovirus-mediated gene transfer to human breast tumor cells: An approach for cancer gene therapy and bone marrow purging." CANCER RESEARCH, vol. 56, no. 6, 1996, pages 1346-1351, XP002112678
- WU A G ET AL: "Bone marrow purging of neuroblastoma by attenuated multimutated herpes simplex virus." PROCEEDINGS OF THE AMERICAN ASSOCIATION FOR CANCER RESEARCH ANNUAL, vol. 39, March 1998 (1998-03), page 605 XP002187903 89th Annual Meeting of the American Association for Cancer Research; New Orleans, USA; 28 March-1 April 1998
- WU A ET AL: "Biological purging of breast cancer cells using an attenuated replication-competent herpes simplex virus in human hematopoietic stem cell transplantation." CANCER RESEARCH, vol. 61, no. 7, 1 April 2001 (2001-04-01), pages 3009-3015, XP002187904
- STOJDL D F ET AL: "Exploiting tumor-specific defects in the interferon pathway with a previously unknown oncolytic virus." NATURE MEDICINE, vol. 6, no. 7, July 2000 (2000-07), pages 821-825, XP002162486
- LICHTY B D ET AL: "Identification of vesicular stomatitis virus as a leukemolytic agent." BLOOD, vol. 96, no. 11 Part 2, 16 November 2000 (2000-11-16), page 213b XP002187915 42nd Annual Meeting of the American Society of Hematology; San Francisco, USA; 1-5 December 2000
- WU A ET AL: "Biological purging of breast cancer cells using an attenuated replication-competent herpes simplex virus in human hematopoietic stem cell transplantation", CANCER RESEARCH, AMERICAN ASSOCIATION FOR CANCER REREARCH, US, vol. 61, no. 7, 1 April 2001 (2001-04-01), pages 3009-3015, XP002187904, ISSN: 0008-5472
- LICHTY B D ET AL: "Identification of vesicular stomatitis virus as a leukemolytic agent", BLOOD, AMERICAN SOCIETY OF HEMATOLOGY, US, vol. 96, no. 11 Part 2, 16 November 2000 (2000-11-16), page 213b, XP002187915, ISSN: 0006-4971
- LILLO R ET AL.: "Efficient and nontoxic adnoviral purging method for autologous transplantation in breast cancer patients", CANCER RESEARCH, vol. 62, 1 September 2002 (2002-09-01), pages 5013-5018,
- ADACHI Y ET AL.: "A midkine promoter-based conditionally replicative adenovirus for treatment of pediatric solid tumors and bone marrow tumor purging", CANCER RESEARCH, vol. 61, 1 November 2001 (2001-11-01), pages 7882-7888,
- GARCIA-CASTRO J ET AL.: "Purging of leukemia-contaminated bone marrow grafts using suicide adenoviral vectors: an in vitro murine experimental model", GENE THERAPY, vol. 10, 2003, pages 1328-1335,
- BEATTIE E ET AL: "Host-range restriction of vaccinia virus E3L-specific deletion mutants.", VIRUS GENES, vol. 12, no. 1, 1996, pages 89-94, ISSN: 0920-8569
- BHAT R A ET AL: "Structural requirements of adenovirus VAI RNA for its translation enhancement function.", MOLECULAR AND CELLULAR BIOLOGY, vol. 5, no. 1, January 1985 (1985-01), pages 187-196, ISSN: 0270-7306
- CASCALLÓ M ET AL: "Ras-dependent oncolysis with an adenovirus VAI mutant.", CANCER RESEARCH, vol. 63, no. 17, 1 September 2003 (2003-09-01), pages 5544-5550, ISSN: 0008-5472
- LILLO R. ET AL: 'Efficient and nontoxic adnoviral purging method for autologous transplantation in breast cancer patients' CANCER RESEARCH vol. 62, 01 September 2002, pages 5013 - 5018
- ADACHI Y. ET AL: 'A midkine promoter-based conditionally replicative adenovirus for treatment of pediatric solid tumors and bone marrow tumor purging' CANCER RESEARCH vol. 61, 01 November 2001, pages 7882 - 7888
- GARCIA-CASTRO J. ET AL: 'Purging of leukemia-contaminated bone marrow grafts using suicide adenoviral vectors: an in vitro murine experimental model' GENE THERAPY vol. 10, 2003, pages 1328 - 1335

## Description

### FIELD OF THE INVENTION

The present invention relates to a method of selectively removing ras-activated neoplastic cells from a mixed cellular composition outside of a living organism by using a virus which selectively infects and kills the neoplastic cells.

### REFERENCES

U.S. Patent No. 6,136,307.
WO 94/18992, published September 1, 1994.
WO 94/25627, published November 10, 1994.
WO 99/08692, published February 25, 1999.
Bar-Eli, N., et al., "preferential cytotoxic effect of Newcastle disease virus on lymphoma cells", J. Cancer Res. Clin. Oncol. 122: 409-415 (1996).
Bensinger, W.I., "Should we purge?", Bone Marrow Transplant. 21:113-115 (1998).
Bischoff JR. et al., "An Adenovirus Mutant that Replicates Selectively in p53-Deficient Human Tumor", Science 274(5286):373-6 (1996).
Blagoslelonny, M.V., et al., "in vitro Evaluation of a p53-Expressing Adenovirus as an Anti-Cancer Drug", Int. J. Cancer 67(3):386-392 (1996).
Bos, J.L.,"Ras Oncogenes in Human Cancer: A Review", Canc. Res. 49(17): 4682-4689 (1989).
Brooks et al., eds. "Jawetz, Melnick & Adelberg's Med. Microbiology". (1998).
Chang et al., PNAS 89:4825-4829 (1992).
Chang, H.W. et al., Virology 194:537-547 (1993).
Chang et al., J. Virol. 69:6605-6608 (1995).
Coffey, M.C., et al., "Reovirus Therapy of Tumors with Activated Ras Pathway", Science 282:1332-1334 (1998).
Duggan, P.R., et al., "Predictive factors for long-term engraftment of autologous blood stem cells", Bone Marrow Transplantation 26(12): 1299-1304 (2000).
Fueyo, J., et al., "A Mutant Oncolytic Adenovirus Targeting the Rb Pathway Produces Anti-Glioma Effect in Vivo", Oncogene 19(1):2-12 (2000).
Gao, J., B. Tombal and J.T. Isaacs, "Rapid in situ hybridization technique for detecting malignant mouse cell contamination in human xenograft tissue from nude mice and in vitro cultures from such xenografts", Prostate 39(1): 67-70 (1999).
Haig, D.M., et al., Immunology 17:4146-4158 (1997).
He, B., et al., Proc. Nat. Acad. Sci. 94: 843-848 (1997).
Kawagishi-Kobayashi, M., et al., Mol. Cell. Biology 17:4146-4158 (1997).
Nemunaitis, J., Invest. New Drugs 17:375-386 (1999).
Nielsen LL., et al., "P53 Tumor Suppressor Gene Therapy for Cancer", Cancer Gene Ther. 5(1):52-63 (1998).
Nieto, Y. et al., "Autologous stem-cell transplantation for solid tumors in adults", Hematol. Oncol. Clin. North Am. 13(5):939-968 (1999).
Norman, K., et al., "Reovirus as a novel oncolytic agent", J. Clin. Invest. 105 (8): 1035-1038 (2000).
Reichard, K.W., et al., "Newcastle Disease Virus Selectively Kills Human Tumor Cells", J. of Surgical Research 52:448-453 (1992).
Stojdl, D.F., et al., "Exploiting Tumor-Specific Defects in the Interferon Pathway with a Previously Unknown Oncolytic Virus", Nat. Med 6(7):821-825 (2000).
Romano et al., Mol. and Cell. Bio. 18:7304-7316 (1998).
Sharp et al., Virol. 250:301-315 (1998).
Spyridonidis, A. et al., "Minimal residual disease in autologous hematopoietic harvests from breast cancer patients", Annals of Onc. 9:821-826 (1998).
Steele, T.A., "Recent Developments in the Virus Therapy of Cancer", Proc. Soc.Exp. BioL Med.. 223:118-127 (2000).
Stewart, D.A., et al., "Superior autologous blood stem cell mobilization from dose-intensive cyclophosphamide, etoposide, cisplatin plus G-CSF than from less intensive chemotherapy regimens", Bone Marrow Transplant. 23(2): 111-117 (1999).
Strong, J.E., et al., "The Molecular Basis of Viral Oncolysis: Usurpation of the Ras Signaling Pathway by Reovirus", EMBO J. 17:3351-3362 (1998).
Strong, J.E., et al., "Minimal Residual Disease in Autologous Hematopietic Harvests from Breast Cancer Patients", Annals of Onc. 9:821-826 (1998).
Strong, J.E., et al., "Evidence that the Epidermal Growth Factor Receptor on Host Cells Confers Reovirus Infection Efficiency", Virology 197(1):405-411 (1993).
Strong, J.E., et al., "The v-erbV oncogene confers enhanced cellular susceptibility to reovirus infection", J. Virol. 70:612-616 (1996).
Wiman KG , "New p53-Based Anti-Cancer Therapeutic Strategies", Med Oncol. 15(4):222-8 (1998).
Winter, J.N., "High-dose therapy with stem-cell transplantation in the malignant lymphomas", Onc. (Huntingt) 13(12):1635-1645 (1999).
Yoon, S.S., et al., "An Oncolytic Herpes Simplex Virus Type I Selectively Destroys Diffuse Liver Metastases from Colon Carcinoma", FASEB J.. 14:301-311(2000).
Zorn, U. et al:, "Induction of Cytokines and Cytotoxicity against Tumor Cells by Newcastle Disease Virus", CancerBiotherapy 9(3):22-235 (1994).

### BACKGROUND OF THE INVENTION

Cell proliferation is regulated by both growth-promoting signals and growth-constraining signals. These two kinds of signals for each cell would normally strike a balance in a manner which reflects the need of the body for the particular cell. If a cell fails to respond to the growth-constraining signals or over-responds to the growth-promoting signals, it will proliferate abnormally fast (referred to as neoplastic cells) and may eventually develop into cancer, a malignant neoplasm.

Chemotherapy, a current method of treating cancer, is generally based on the fast-proliferating property of cancer cells. Since cancer cells proliferate rapidly, they are more sensitive to drugs which inhibit cellular proliferation. In theory, by carefully choosing the dosage of chemotherapeutic drugs, one can inhibit cancer cell proliferation without seriously damaging normal cells. However, some normal cells, such as hematopoietic stem cells, also proliferate rapidly. Therefore, any dosage which is harmful to cancer cells is often also harmful to the hematopoietic stem cells. On the other hand, if the dosage is not high enough to kill the cancer cells, there is a risk that the cancer would reappear shortly after chemotherapy is terminated.

Because it is hard to find a dosage which selectively kills cancer cells, high-dose chemotherapy followed by autologous hematopoietic progenitor stem cell transplantation has gained extensive application as a therapeutic approach in many cancers (for example, see Winter, 1999; Nieto and Shpall, 1999). In this approach, a portion of the hematopoietic stem cells is removed from a cancer patient, and the patient is then treated with high-dose chemotherapy which is lethal to rapid-proliferating cells, such as cancer cells and hematopoietic stem cells. Subsequently, the patient receives transplantation of autologous hematopoietic stem cells, which have been previously removed from the same patient, to regenerate the hematopoietic system.

A serious drawback of this therapy is that when the hematopoietic progenitor stem cells are removed from the patients, they are often contaminated with cancer cells. This is especially a problem when the patient has a cancer of hematopoietic origin, but patients with a solid tumor may also suffer from contamination of the hematopoietic stem cells, particularly if the solid tumor has metastasized. As a result, when the removed cells are transplanted back to reestablish the hematopoietic system, some cancer cells may also be placed back to the cancer patient where they may proliferate again to contribute to cancer recurrence. It is therefore desirable to purge the autografts before transplantation.

Several methods have been employed to purge autografts (Spyridonidis et al, 1998; Bensinger 1998). The autograft can be treated with chemotherapy to kill the contaminating neoplastic cells *in vitro.* However, as discussed above, it is hard to find a dosage for the chemotherapeutic drug which selectively kills neoplastic cells or cancer cells but leaves normal hematopoietic stem cells intact. Autografts can also be treated with a toxin conjugated to antibodies which recognize an antigen that is specific for the neoplastic cells, but such a tumor specific antigen does not always exist. It is also possible to separate stem cells from the other cells based on a stem cell specific surface marker (CD34) by using flow cytometry, affinity columns or magnetic beads. However, by selecting only certain hematopoietic cells, e.g., the CD34⁺ cells, other hematopoietic cells such as T cells, B cells, monocytes and natural killer cells are also eliminated, and immune recovery may be delayed (Bensinger, 1998). This method also results in the loss of about half the CD34⁺ cells and retention of some contaminating cancer cells (Spyridonidis et al., 1998).

Therefore, there remains a need for a highly selective method with a reasonable yield to purge autografts which may contain neoplastic cells.

### SUMMARY OF THE INVENTION

The present invention is defined in and by the appended claims. The invention is directed method of selectively removing ras-activated neoplastic cells from a mixed cellular composition, for example an autograft, by using a virus which exhibits selective killing of neoplastic cells.

Accordingly, one aspect of the present invention is directed to a method of selectively removing ras-activated neoplastic cells from a mixed cellular composition suspected of containing neoplastic cells as defined in claim 1.

In an embodiment of the invention, the method further comprises the step of freezing and storing the virus-treated cellular composition in a solution containing DMSO. DMSO is routinely used to freeze and store animal cells but it can denature viruses. Therefore, DMSO treatment removes infectious virus from the cellular composition while preserving the activity of the composition in the frozen state for a prolonged period of time.

In another embodiment of the present invention, the virus is removed from the virus-treated cellular composition by subjecting the mixture to anti-virus antibodies which are specific for the particular virus, or a combination of anti-virus antibodies and complement in order to lyse the virus. Alternatively or additionally, anti-virus antibodies which recognize a molecule on the surface of the virus particle may be used to remove the virus particles by immobilizing the antibodies, applying the cellular composition to the immobilzed antibodies, and collecting the part of the composition which does not bind to the antibodies.

Similarly, specific antibodies against the particular virus can be administered to the transplant recipient to eliminate the virus *in vivo*, or the recipient can be given an immune system stimulant to achieve this purpose.

In another embodiment of the present invention, the virus is removed from the virus-treated cellular composition by using a gradient which can separate viruses from cells.

In a preferred embodiment of this invention, the mixed cellular composition comprises hematopoietic stem cells. Thus, hematopoietic stem cells can be purged prior to transplantation, or any other desired use, to remove the neoplastic cells. The hematopoietic stem cells can be harvested from bone marrow or blood.

The application of this invention is not limited to purging hematopoietic stem cells. In another embodiment of this invention, the present method can be applied to any tissue, organ, a combination of different tissues/organs, or any portion of a tissue or an organ to remove neoplastic cells. The tissues or organs are preferably useful in a subsequent transplantation. However, the present method is also useful in purging tissues or organs for any other purposes wherein it is desirable to remove neoplastic cells which are present in the tissue or organ.

In another embodiment of the invention, the virus is used to treat cultured cell lines to remove cells which are spontaneously transformed. This method can also be used to treat semen or donor eggs before artificial insemination or other reproduction-related procedures.

In another aspect of this invention, the virus is a replication competent virus. As opposed to a replication-deficient virus, a replication competent virus can replicate in a cell which is susceptible to this virus and often causes this cell to lyse. The replication competent virus useful in this invention can selectively lyse neoplastic cells in a phenomenon termed "oncolysis", but it does not lyse normal cells.

In another embodiment of this invention, the virus is a mutated virus as defined in claim 1. Each of these viruses in the native form has developed a mechanism to inhibit the double stranded RNA protein kinase (PKR) to facilitate viral protein synthesis which is otherwise inhibited by PKR. These viruses can therefore replicate in any cells regardless of PKR. When these viral PKR inhibitors are mutated or modified, however, the virus is then susceptible to PKR inhibition and does not replicate in normal cells, which have a functional PKR pathway. These mutated or modified viruses can be used to selectively remove ras-activated neoplastic cells because ras-activated neoplastic cells are deficient in PKR function and thus can not inhibit replication of these viruses.

Also described herein are cellular compositions which have been treated with a virus to remove neoplastic cells and leave viable non-neoplastic cells. Such compositions may be used for *in vitro* research, or in transplantation, insemination, or other *in vivo* procedures. The transplantation may be autologous, allogeneic, or even xenogeneic. Preferably the transplantation is autologous. More preferably, the composition comprises hematopoietic stem cells.

### BRIEF DESCRIPTION OF THE DRAWINGS

Figure 1

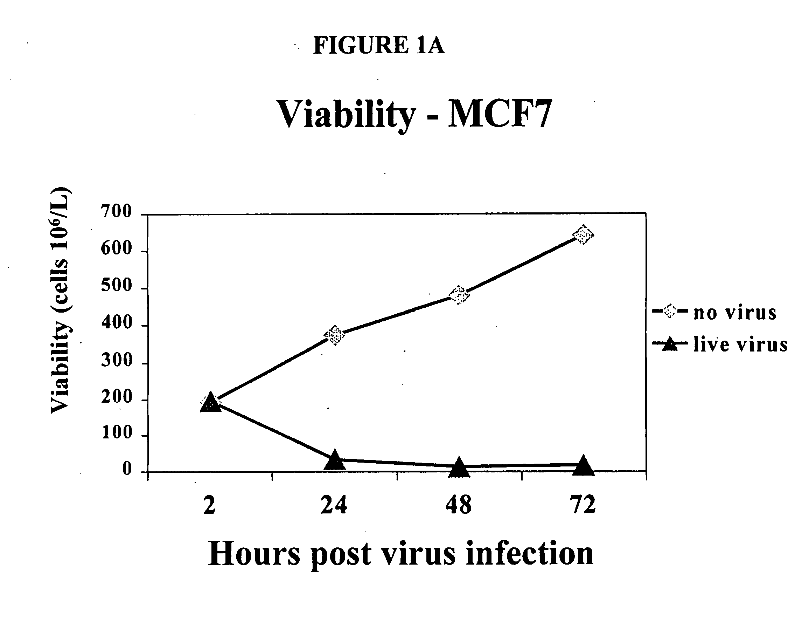

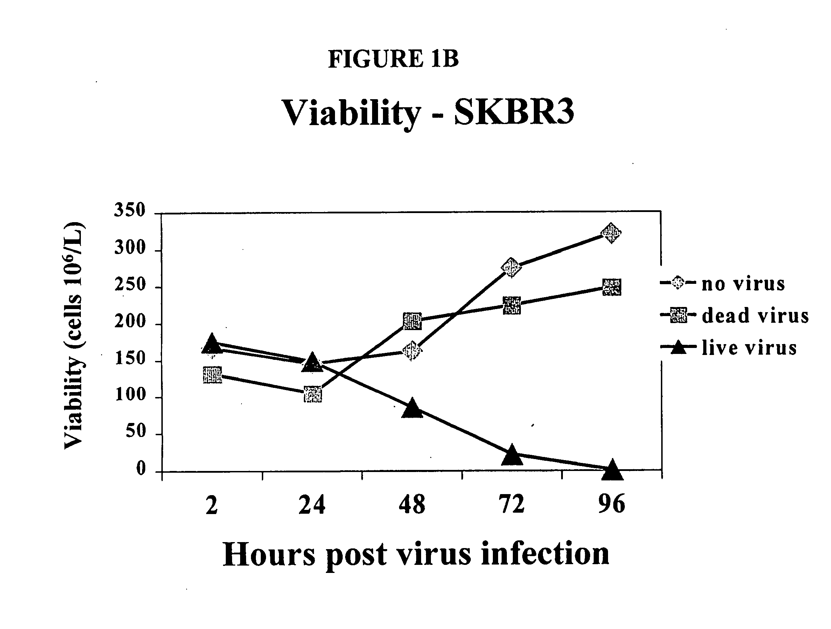

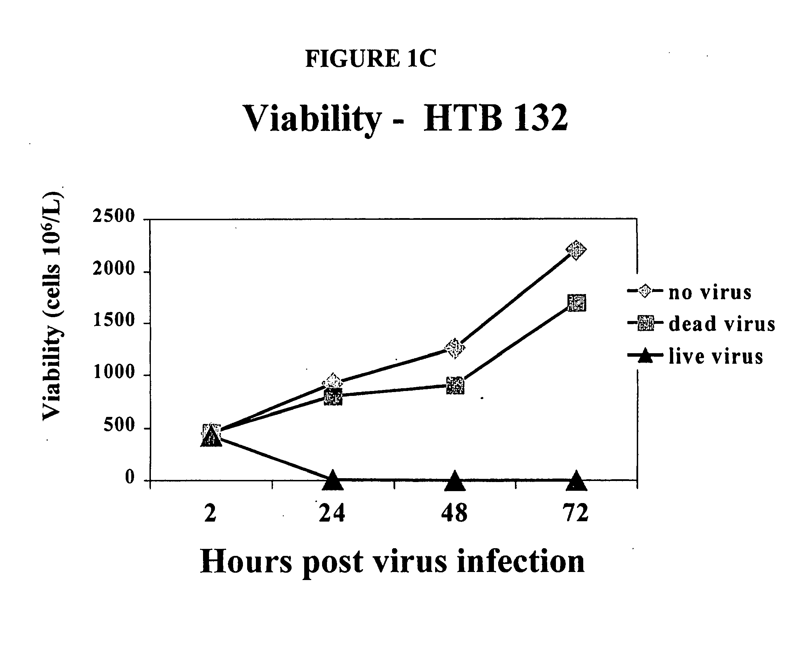

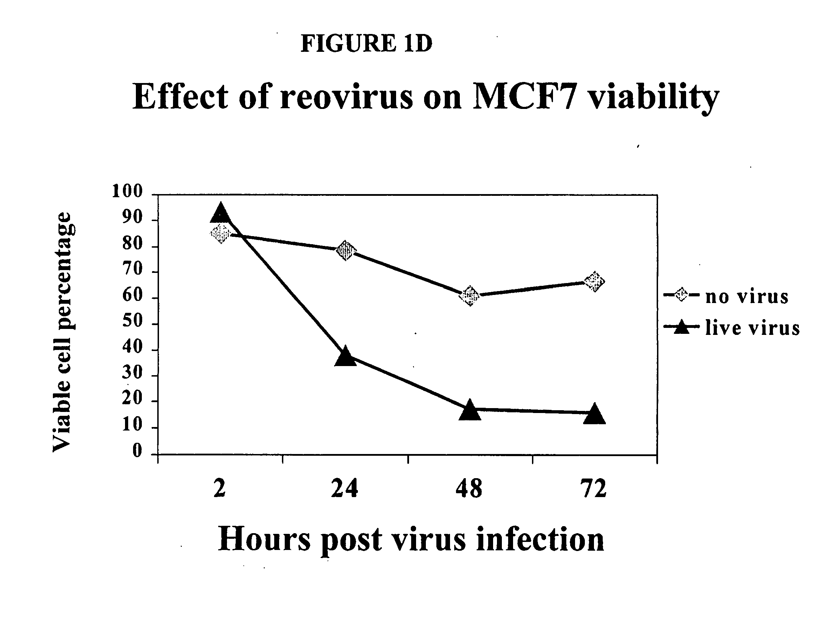

   Figures 1A-1C show the number of viable cells in MCF7 (Figure 1A), SKBR3 (Figure 1B) or HTB 132 (Figure 1C) which were infected with live reovirus, dead virus or no virus as indicated. Figure 1D shows the percentage of MCF7 cells which were viable at various time points after reovirus infection.
Figure 2

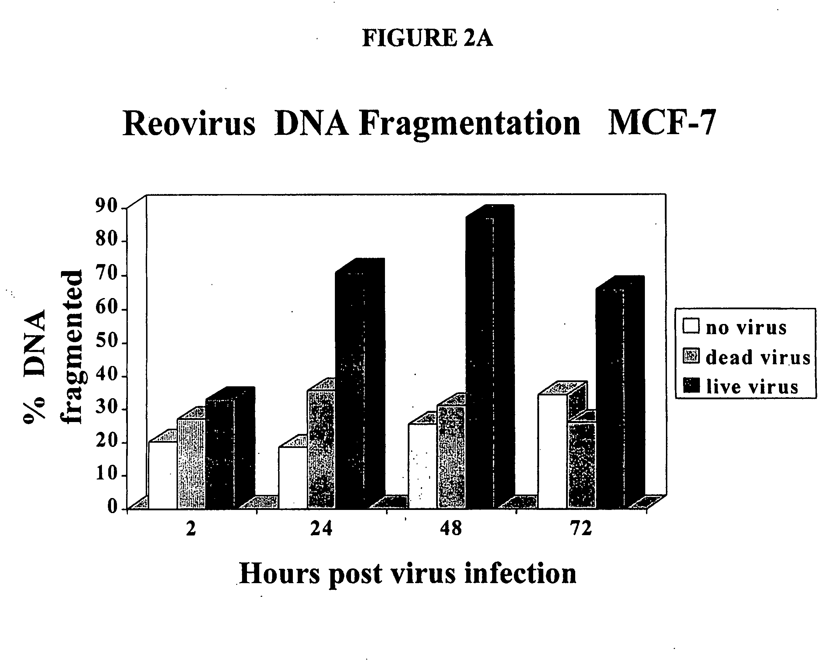

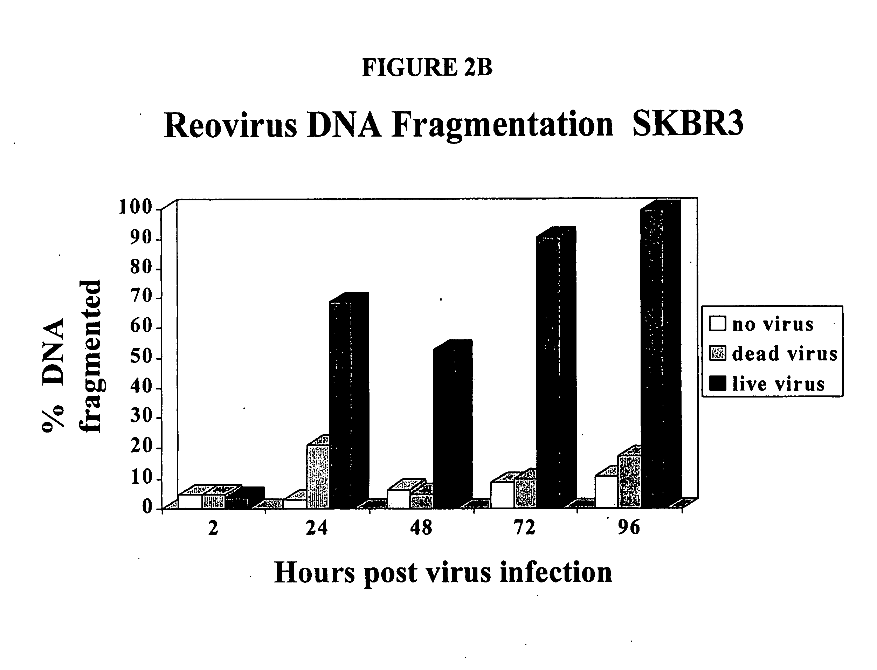

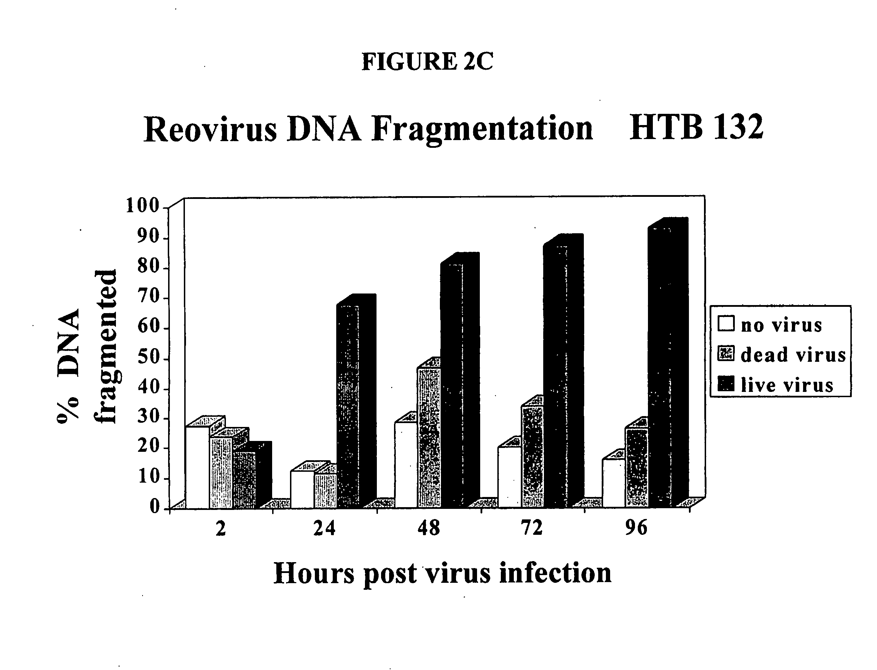

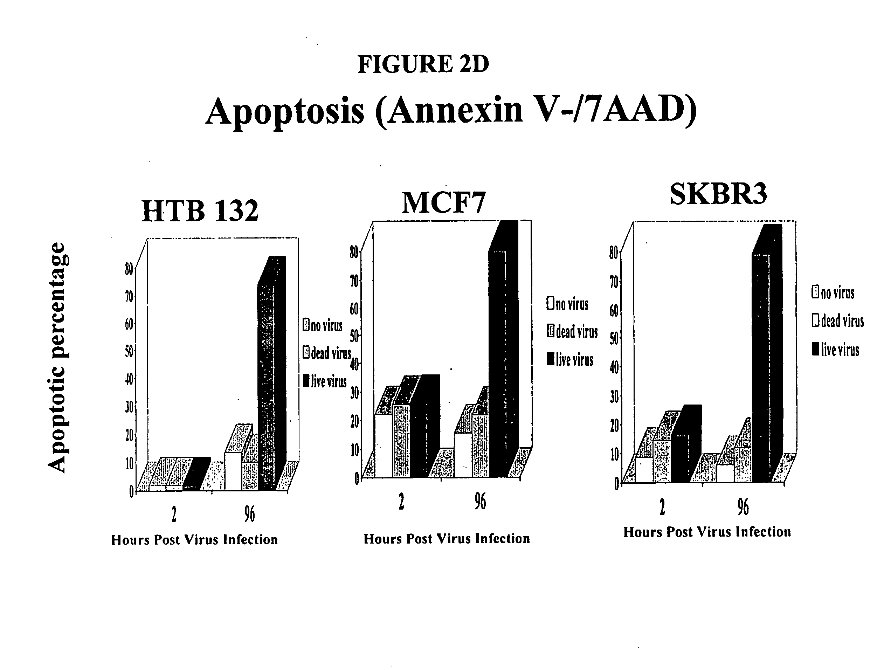

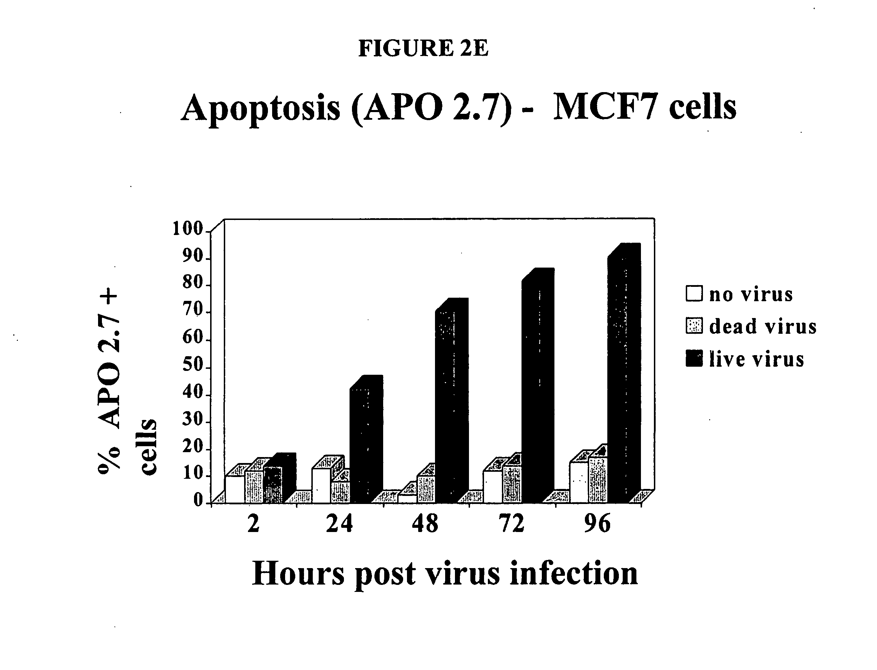

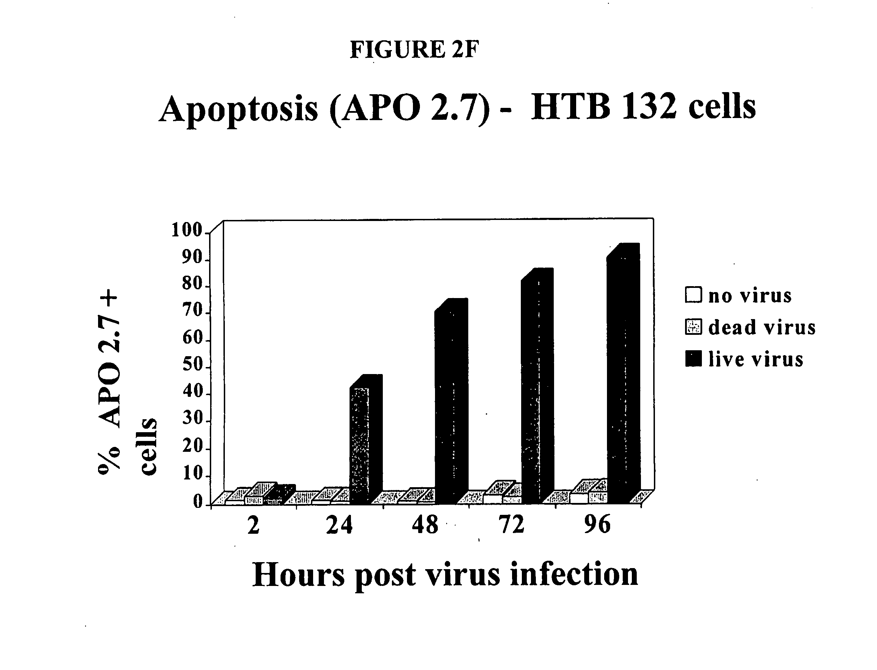

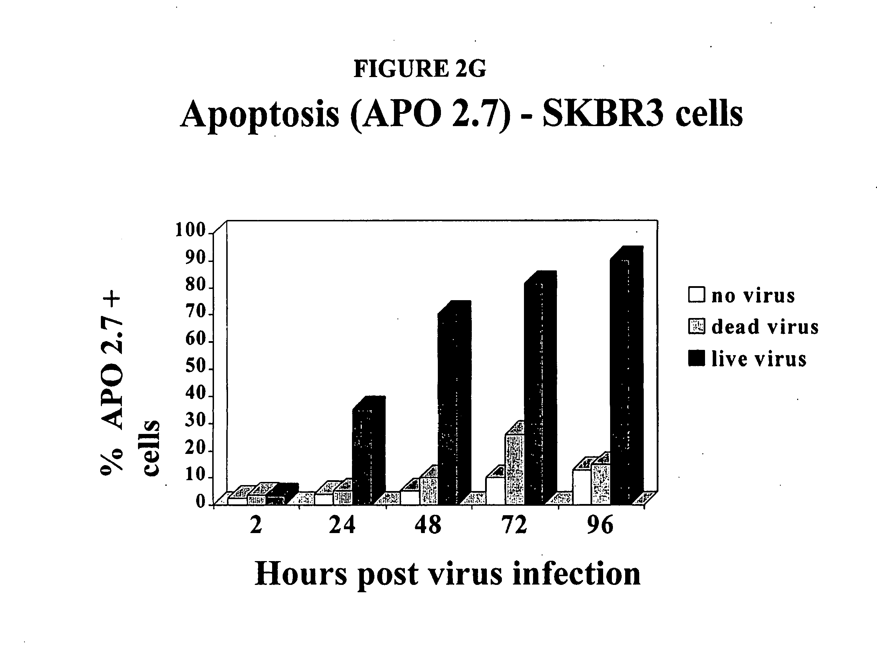

   Figure 2 shows that apoptosis was induced by reovirus infection in MCF7, SKBR3 or HTB 132 cells. Figures 2A-2C demonstrate the percentage DNA which were fragmented after reovirus infection. Figure 2D shows the percentage of the apoptotic marker Annexin V staining after reovirus infection. Figures 2E-2G show the percentage of AP02.7⁺ cells in each cell type as indicated.
Figure 3

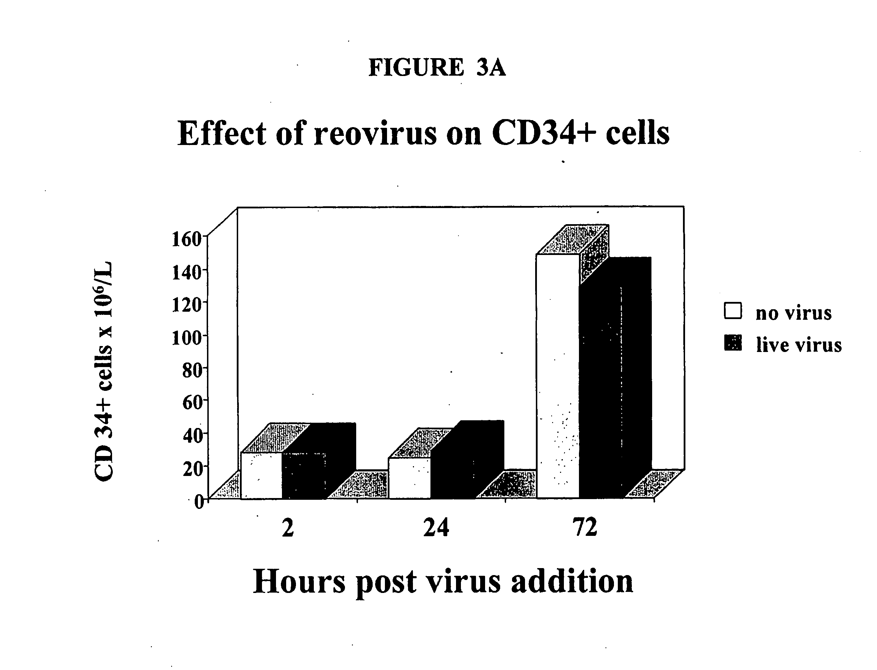

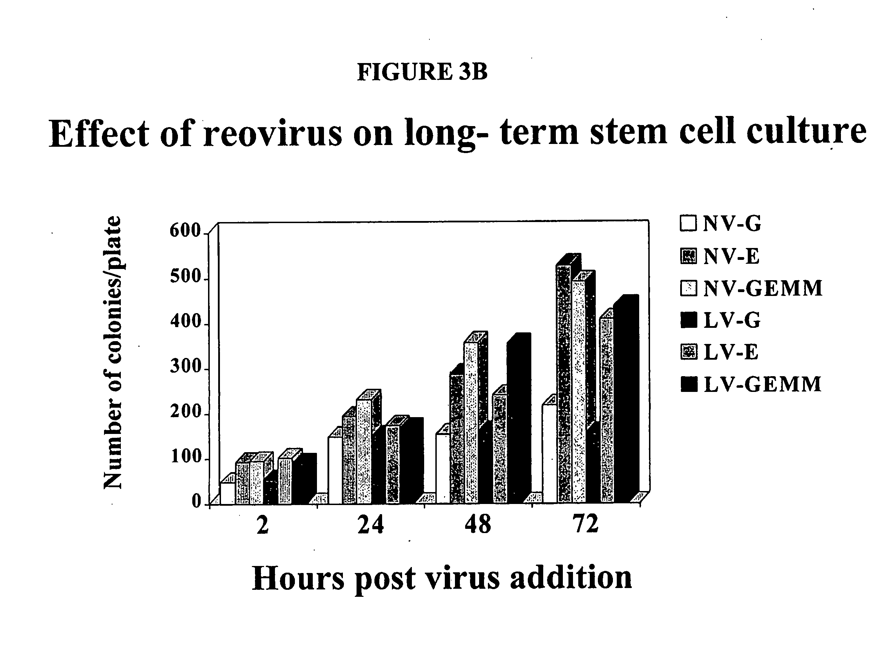

   Figure 3A shows the number of viable cells at various time points after CD34⁺ stem cells had been infected with reovirus. Figure 3B shows the effect of reovirus on long-term stem cell culture. Stem cells were infected with reovirus and incubated for 2, 24, 48 or 72 hours, respectively, then the cells were diluted and cultured for 14 days to allow individual colonies to form. The number of each kind of colony, granulocytes (G), erythroids (E) or granulocyte erythroid macrophage megakaryocyte (GEMM), was then determined for cells infected with no virus (NV) or live virus (LV), respectively. For example, NV-G stands for the granulocyte colonies derived from cells which were treated with no virus, and LV-G stands for those derived from cells which were treated with live reovirus.
Figure 4

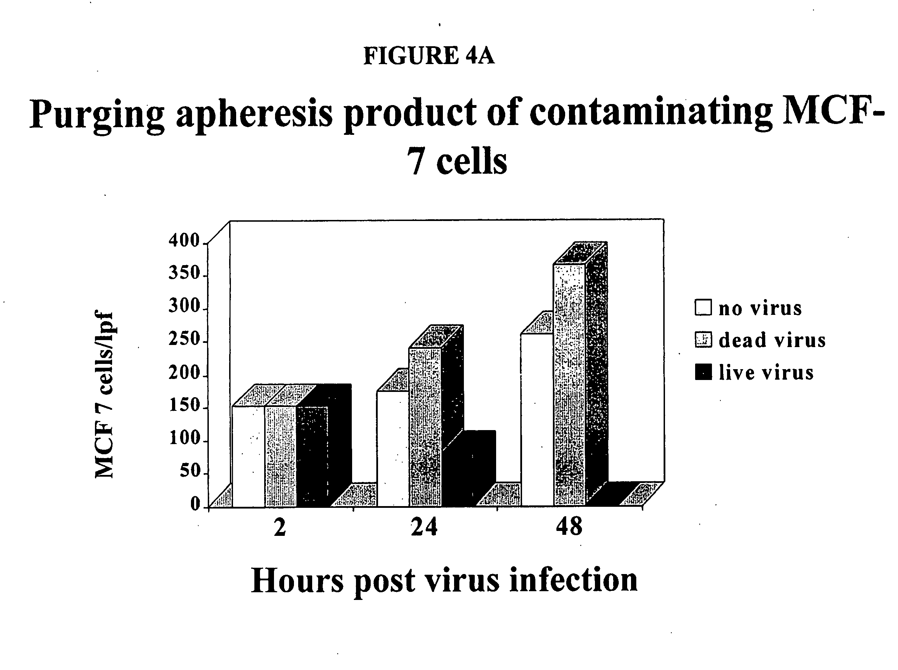

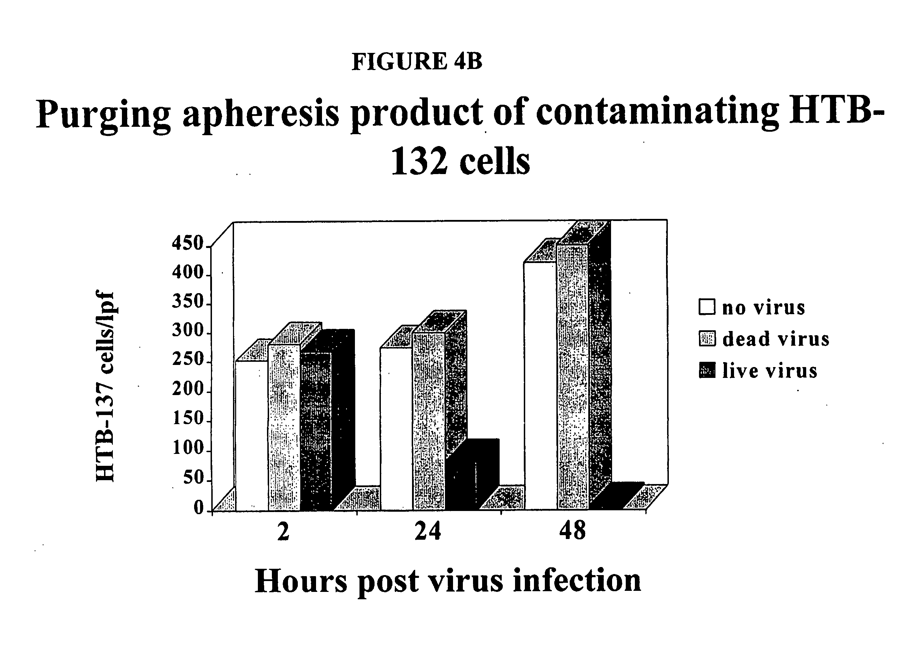

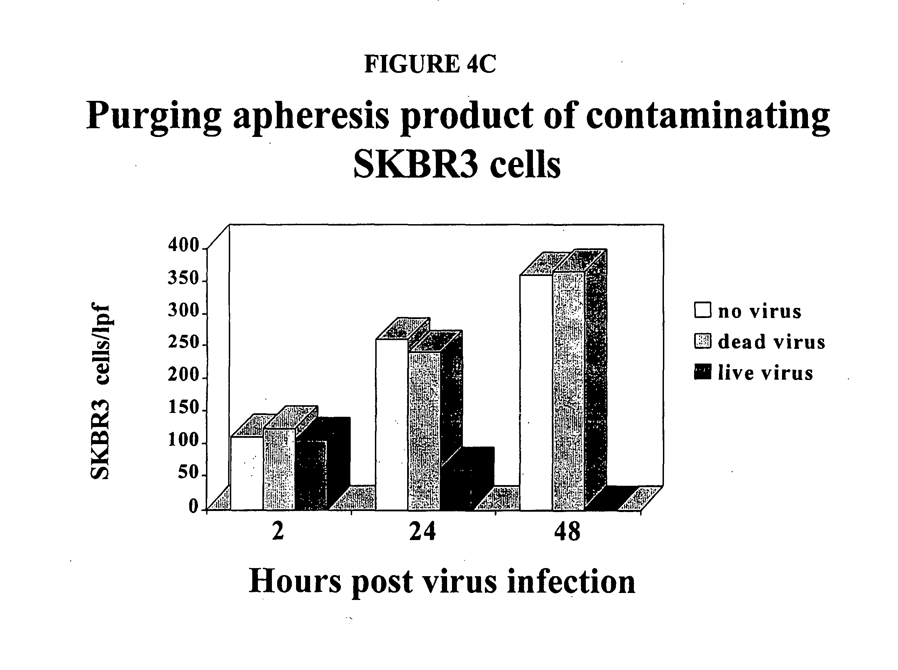

   Figures 4A-4C show the purging effects of reovirus on mixtures of apheresis product with MCF7, MDA MB 468 or SKBR3 cells, respectively.

### DETAILED DESCRIPTION OF THE INVENTION

The present invention is directed to a method for selectively removing ras-activated neoplastic cells from a mixed cellular composition, for example an autograft, by using a virus (as defined in claim 1) which exhibits selective killing of neoplastic cells.

Prior to describing the invention in further detail, the terms used in this description are defined as follows unless otherwise indicated.

### Definitions

"Virus" refers to any virus, whether in the native form, attenuated or modified. Modified viruses include chemically modified viruses or recombinantly modified viruses. A recombinantly modified virus may be a mutated virus, a recombinant virus or a reassorted virus. A mutated virus is a virus in which the viral genome has been mutated, namely having nucleotide insertions, deletions and/or substitutions. A recombinant virus is a virus having coat proteins from different subtypes, usually prepared by co-infecting a cell with more than one subtype of the virus, resulting in viruses which are enveloped by coat proteins encoded by different subtypes. A reassorted virus is a multi-segment virus in which the segments have been reassorted, usually by co-infecting a cell with more than one subtype of this virus so that the segments from different subtypes mix and match in the cell.

"Neoplastic cells", also known as "cells with a proliferative disorder", refer to cells which proliferate without the normal growth inhibition properties. A new growth comprising neoplastic cells is a neoplasm or tumor. A neoplasm is an abnormal tissue growth, generally forming a distinct mass, which grows by cellular proliferation more rapidly than normal tissue growth. Neoplasms may show partial or total lack of structural organization and functional coordination with normal tissue. As used herein, a neoplasm is intended to encompass hematopoietic neoplasms as well as solid neoplasms.

A neoplasm may be benign (benign tumor) or malignant (malignant tumor or cancer). Malignant tumors can be broadly classified into three major types. Malignant neoplasms arising from epithelial structures are called carcinomas, malignant neoplasms that originate from connective tissues such as muscle, cartilage, fat or bone are called sarcomas and malignant tumors affecting hematopoietic structures (structures pertaining to the formation of blood cells) including components of the immune system, are called leukemias and lymphomas. Other neoplasms include, but are not limited to neurofibromatosis.

"Ras-activated neoplastic cells" or "ras-mediated neoplastic cells" refer to cells which proliferate at an abnormally high rate due to, at least in part, activation of the ras pathway. The ras pathway may be activated by way of ras gene structural mutation, elevated level of ras gene expression, elevated stability of the ras gene message, or any mutation or other mechanism which leads to the activation of ras or a factor or factors downstream or upstream from ras in the ras pathway, thereby increasing the ras pathway activity. For example, activation of EGF receptor, PDGF receptor or Sos results in activation of the ras pathway. Ras-mediated neoplastic cells include, but are not limited to, ras-mediated cancer cells, which are cells proliferating in a malignant manner due to activation of the ras pathway.

"Cellular composition" means a composition comprising cells. The composition may contain non-cellular matter. For example, whole blood is a cellular composition which contains plasma, platelets, hormones and other non-cellular matter in addition to cells such as erythrocytes and leukocytes. A cellular composition may contain cells of various types, origin or organization. For example, tissues and organs which contain different cell types arranged in defined structures are considered cellular compositions.

A "mixed cellular composition" is a cellular composition containing at least two kinds of cells. Typically, the mixed cellular composition contains both normal cells and neoplastic cells. It is preferable that most of the cells in the cellular composition are dividing cells, and the virus selectively kills neoplastic cells but leaves other dividing cells essentially intact.

A cellular composition "suspected of containing neoplastic cells" is a cellular composition which may contain neoplastic cells. For example, any autograft obtained from a subject bearing a neoplasm may contain neoplastic cells. A cell culture which has been in culture for a considerable amount of time may contain spontaneous by neoplastic cells.

"Substantial killing" means a decrease of at least about 20% in viability of the target neoplastic cells. The viability can be determined by a viable cell count of the treated cells, and the extent of decrease can be determined by comparing the number of viable cells in the treated cells to that in the untreated cells, or by comparing the viable cell count before and after virus treatment. The decrease in viability is preferably at least about 50%, more preferably at least about 70%, still more preferably at least about 80%, and most preferably at least about 90%.

The neoplastic cells may be killed in various manners. For example, they may be lysed by a virus which is capable of lytic infection of neoplastic cells (oncolysis). The neoplastic cells may undergo apoptosis which is induced directly or indirectly by the virus. The cells may also, although less preferably, be killed by the immune system which has been activated by the virus. For example, the virus may induce cytokine production, which activates the natural killer cells, which in turn selectively kills neoplastic cells (Zorn et al., 1994).

A "replication competent" virus is a virus which is capable of replicating in at least one cell type. As opposed to a replication competent virus, a "replication incompetent virus" contains a mutation in a region of its genome which is essential for its replication, and hence is not capable of replicating in any cell.

"Adenovirus" is a double stranded DNA virus of about 3.6 kilobases. In humans, adenoviruses can replicate and cause disease in the eye and in the respiratory, gastrointestinal and urinary tracts. About one-third of the 47 known human serotypes are responsible for most cases of human adenovirus disease (Brooks et al., 1998).

The term "mutated adenovirus" or "modified adenovirus" means, as used herein, that the gene product or products which prevent the activation of PKR are lacking, inhibited or mutated such that PKR activation is not blocked. The adenovirus encodes several gene products that counter antiviral host defense mechanisms. The virus-associated RNA (VAI RNA or VA RNA₁) of the adenovirus are small, structured RNAs that accumulate in high concentrations in the cytoplasm at late time after adenovirus infection. These VAI RNA bind to the double stranded RNA (dsRNA) binding motifs of PKR and block the dsRNA-dependent activation of PKR by autophosphorylation. Thus, PKR is not able to function and the virus can replicate within the cell. The overproduction of virons eventually leads to cell death. In a mutated or modified adenovirus, the VAI RNA's are preferably not transcribed. Such mutated or modified adenovirus would not be able to replicate in normal cells that do not have an activated Ras-pathway; however, it would be able to infect and replicate in cells having an activated Ras-pathway.

"Vaccinia virus" refers to the virus of the orthopoxvirus genus that infects humans and produces localized lesions (Brooks et al., 1998). Vaccinia virus encodes two genes that play a role in the down regulation of PKR activity through two entirely different mechanisms. E3L gene encodes two proteins of 20 and 25 kDa that are expressed early in infection and have dsRNA binding activity that can inhibit PKR activity. Deletion or disruption of the E3L gene creates permissive viral replication in cells having an activated Ras pathway. The K3L gene of vaccinia virus encodes pK3, a pseudosubstrate of PKR.

The term "mutated vaccinia virus" or "modified vaccinia virus" means, as used herein, that the gene product or products which prevent the activation of PKR are lacking, inhibited or mutated such that PKR activation is not blocked. Preferably, the E3L gene and/or the K3L gene is not transcribed. Such mutated or modified vaccinia virus would not be able to replicate in normal cells that do not have an activated Ras-pathway, however, it would be able to infect and replicate in cells having an activated Ras-pathway.

"Resistance" of cells to viral infection means that infection of the cells with the virus does not result in significant viral production or yield.

A "viral oncolysate" is a composition prepared by treating tumor cells with an oncolytic virus *in vitro,* which composition is subsequently administered to a tumor patient with the same kind of tumor in order to induce immunity in the tumor patient against this tumor. As such, viral oncolysates are essentially virus-modified cancer cell membranes.

As used herein, a "transplant recipient" is a mammal which receives a transplantation of cellular compositions. Preferably the recipient is a human, and more preferably the recipient is a human who is receiving transplantation in the treatment of cancer.

### Method

The present invention relates to the use of a virus (as defined in claim 1) to selectively remove ras-activated neoplastic cells from mixed cellular compositions which are suspected of containing neoplastic cells. Although reovirus is used as an example below, a person of ordinary skill in the art can follow the instructions herein as and apply the method to purge any mixed cellular composition by using viruses as defined in claim 1. The use of reovirus is not in accordance with the claimed invention.

### 1. Reovirus

We recently discovered that reovirus selectively lyses ras activated neoplastic cells *in vitro, in vivo* and *ex vivo* (Coffey et al., 1998; WO 99/08692). Normally, cells are not susceptible to reovirus infection. However, if the ras pathway is activated, reovirus can successfully replicate in the cells and eventually results in lysis of the host cells. For example, when reovirus-resistant NIH 3T3 cells were transformed with activated Ras or Sos, a protein which activates Ras, reovirus infection was enhanced (Strong et al., 1998). Similarly, mouse fibroblasts that are resistant to reovirus infection became susceptible after transfection with the EGF receptor gene or the v-erbB oncogene (Strong et al., 1993; Strong et al., 1996).

Without being limited to a theory, it seems that reovirus replication is regulated at the translational level (Strong et al., 1998; Norman et al., 2000). In untransformed NIH 3T3 cells, early viral transcripts activate the double-stranded RNA-activated protein kinase (PKR), which inhibits translation, thereby inhibiting viral replication. Activated Ras (or an activated element of the ras pathway) presumably inhibits or reverses PKR activation. Therefore, viral protein synthesis proceeds, viral particles are made, and the cells are eventually lysed.

The ras oncogene accounts for a large number of tumors. Activating mutations of the ras gene itself occur in about 30% of all human tumors (Bos, J.L.,1989), primarily in pancreatic (90%), sporadic colorectal (50%) and lung (40%) carcinomas, and myeloid leukemia (30%). Activation of the factors upstream or downstream of ras in the ras pathway is also associated with tumors. For example, overexpression of HER2/Neu/ErbB2 or the epidermal growth factor (EGF) receptor is common in breast cancer (25-30%), and overexpression of platelet-derived growth factor (PDGF) receptor or EGF receptor is prevalent in gliomas and glioblastomas (40-50%). EGF receptor and PDGF receptor are both known to activate ras upon binding to their respective ligand, and v-erbB encodes a constitutively activated receptor lacking the extracellular domain.

We first determined the ability of reovirus to kill cancer cells. Reovirus efficiently caused oncolysis of three breast cancer model systems, MCF7, SKBR3 and HTB 132, by inducing apoptosis in the infected cells (Example 1). Thus, reovirus treatment resulted in a marked decrease in viability of MCF7, SKBR3 and HTB 132 cells, while controls treated with no virus or dead virus grew normally (Figures 1A-1D). The decrease in viability was accompanied by characteristics which are associated with apoptosis, such as DNA fragmentation, annexin V or APO 2.7 staining positivity (Figures 2A-2G) and cytopathic effects, such as cell membrane blebbing, nuclear condensation and chromatin condensation observed under the microscope.

Since reovirus infection is usually blocked at the translational level in normal cells but not in ras-mediated neoplastic cells, we examined the extent of protein synthesis in reovirus treated MCF7 cells and CD34⁺ stem cels (Example 2). Indeed, viral proteins were synthesized in the reovirus infected cancer cell line, but not in CD34⁺ stem cells which were also treated with reovirus (data not shown). This result suggests that it will be safe to treat hematopoietic stem cells with reovirus, since reoviral proteins were not synthesized in reovirus treated stem cells and cellular protein synthesis proceeded normally. To confirm this point, viability of the reovirus treated CD34⁺ cells was determined at various time points after reovirus treatment (Example 3). Cell numbers in populations treated with live reovirus or no virus were similar after each time point (Figure 3A), indicating that CD34⁺ cells are not susceptible to reovirus infection.

In order for reovirus to be useful in purging hematopoietic stem cell in high dose chemotherapy treatments, it is essential that the reovirus treatment does not alter the ability of stem cells to differentiate into each and every hematopoietic lineage to reconstitute the whole hematopoietic system. Therefore, long term effect of reovirus treatment was assessed (Example 3). CD34⁺ cells treated with either no virus or live virus showed essentially no difference in their ability to differentiate into granulocytes, erythroids, or granulocyte erythroid macrophage megakaryocytes even after 72 hours of reovirus treatment (Figure 3B). The ratio between these three lineages also remained the same after this prolonged treatment. Accordingly, reovirus treatment neither killed CD34⁺ cells nor changed the potential of them to reconstitute the hematopoietic system.

Furthermore, reovirus is capable of purging a mixed cellular composition, as demonstrated by the selective killing of MCF7, SKBR3 or HTB 132 cells in a mixture of cancer cells and apheresis product which contained CD34⁺ stem cells (Example 4). By measuring CD34 and cytokeratin, a marker specific for epithelial cells such as MCF7, SKBR3 or HTB 132, it was shown that reovirus essentially eliminated the cancer cells from the mixed cellular composition (Figures 4A-4C) while leaving the stem cells intact. Therefore, reovirus treatment is an efficient method to purge neoplastic cells from hematopoietic stem cell compositions.

Accordingly stem cell-containing autografts are treated with reovirus prior to transplantation to remove the contaminating or spontaneous ras-activated neoplastic cells. This increases the efficacy of the high dose chemotherapy/autologous hematopoietic stem cell transplantation treatment. Of particular interest will be the treatment of Hodgkin's disease, multiple myeloma, non-Hodgkin's lymphoma, acute myelogenous leukemia, germ cell (testicular) cancers, brain tumors, and breast tumors, since high dose chemotherapy and autologous stem cell transplantation have been performed efficiently in patients with these tumors. However, it is contemplated that the present method will be useful in other cancers as well to remove any ras-mediated neoplastic cells, since activation of the ras pathway may occur in any cell or tissue type.

Hematopoietic progenitor stem cells can be obtained from the bone marrow of the patient in advance of treatment. Alternatively, in a cancer patient who has been receiving traditional, non-high dose chemotherapy, many stem cells typically appear in the peripheral blood with or without colony stimulating factor priming. Therefore, hematopoietic progenitor stem cell can be obtained from the blood as apheresis product, which can be stored for a long time before being transplanted. The present invention can be applied to stem cell-containing autografts which are harvested from any tissue source, including bone marrow and blood.

In addition to hematopoietic stem cells, the present invention can be broadly applied to remove ras-activated neoplastic cells from many other cellular compositions. For example, the invention can be used as a routine practice to "clean up" (remove ras-activated neoplastic cells from) any tissue or organ transplant. Of particular interest will be the use of the claimed methods to clean up whole blood or any portion thereof for a subsequent transfusion. Similarly, tissue or organ transplantation has become increasingly common, and it will be beneficial if the transplant can be treated to remove ras-activated neoplastic cells before transplantation. Liver, kidney, heart, cornea, skin graft, pancreatic islet cells, bone marrow or any portions thereof are just a few examples of the tissues or organs to which this invention can be applied.

The tissue or organ can be autologous, allogeneic or xenogeneic. The tissue or organ may also be derived from a transgenic animal, be a tissue/organ which is developed *in vitro* from stem cells, or be expanded *ex vivo.* The tissue or organ to be treated can be from an embryonic or adult origin. For example, embryonic neuronal cells can be treated before being transplanted into an Alzheimer's patient. Similarly, the invention can be used to treat semen or donor eggs *ex vivo.*

Application of the present invention is not limited to transplants. Rather, any cellular compositions can be "cleaned up" for any purpose. Thus, all the examples described above are applicable even if the tissue or organ is not meant for transplantation.

Cell lines may also be treated routinely to safeguard against spontaneous or contaminating ras-activated neoplastic cells. Again, any cell line will be a good candidate for this method except, of course, a cell line transformed by means of activation of the ras pathway.

Recently, many laboratories have been attempting to establish serially transplantable xenografts of human prostate cancer tissue inoculated into immune-compromised mice. However, contamination with mouse cancer cells often occurs during the serial passage of the xenografts and these calls can eventually outgrow the human prostate cancer cells (Gao et al., 1999). The present invention will be a simple solution to this problem if the contaminating cancer is ras-mediated and the xenograft is not.

The present invention is distinct from a method of preparing viral oncolysates. Tumor cells are often poor inducers of immune response and can thus escape the attack of the immune system. Viral oncolysates, essentially virus-modified tumor cell membranes, are used in an approach to enhance the immunogenicity of tumor cells. To prepare viral oncolysates, tumor cells are removed from a subject bearing the tumor, and infected with a virus which lyses the tumor cells. The resulting substance is then administered to a subject bearing the tumor, and immunity is often induced against the uninfected tumor cells. The mechanism whereby virus infection of tumor cells induces immunity to uninfected tumor cells is unknown, but virus xenogenization of tumor cells may be involved (Steele, 2000).

Oncolysates of influenza virus-infected melanoma, vulvar carcinoma and ovarian carcinoma, as well as newcastle disease virus infected colon carcinoma oncolysates and vaccinia virus oncolysates have all been used against various tumors. For example, a melanoma patient received oncolysates after surgical excision of the tumor. The viral oncolysate was administered weekly to week 4, every 2 weeks to week 52, every 3 weeks to week 120, and every 6 weeks to week 160. In another clinical case, the administration schedule of autologous NDV oncolysate against colorectal cancer was initiated 2 weeks after surgery and repeated 5 times at 2-week intervals , followed by one boost 3 months later (Nemunaitis, 1999). The studies showed a clinical response in some patients or generation of active immunity against tumor antigens (Steele, 2000).

The present invention is distinct from viral oncolysates in that it is not related to virus-modified tumor cells. In contrast to viral oncolysates, the lysed neoplastic cells can be, and preferably are, removed from the virus-treated cellular composition without affecting the efficacy of the present invention. Furthermore, viral oncolysates are prepared using mostly tumor cells, whereas the mixed cellular composition in the present invention preferably contains less than 60% neoplastic cells, more preferably less than 40%, still more preferably less than 20%, and most preferably less than 10% neoplastic cells.

### 2. Other viruses which selectively kill ras-activated neoplastic cells

Normally, when virus enters a cell, double stranded RNA Kinase (PKR) is activated and blocks protein synthesis, and the virus can not replicate in this cell. Some viruses have developed a system to inhibit PKR and facilitate viral protein synthesis as well as viral replication. For example, adenovirus makes a large amount of a small RNA, VA1 RNA. VA1 RNA has extensive secondary structures and binds to PKR in competition with the double stranded RNA (dsRNA) which normally activates PKR. Since it requires a minimum length of dsRNA to activate PKR, VAI 1RNA does not activate PKR. Instead, it sequesters PKR by virtue of its large amount. Consequently, protein synthesis is not blocked and adenovirus can replicate in the cell.

Vaccinia virus encodes two gene products, K3L and E3L, which down-regulate PKR with different mechanisms. The K3L gene product has limited homology with the N-terminal region of eIF-2α, the natural substrate of PKR, and may act as a pseudosubstrate for PKR. The E3L gene product is a dsRNA-binding protein and apparently functions by sequestering activator dsRNAs.

As discussed above, ras-activated neoplastic cells are not subject to protein synthesis inhibition by PKR, because ras inactivates PKR. These cells are therefore susceptible to viral infection even if the virus does not have a PKR inhibitory system. Accordingly, if the PKR inhibitors in adenovirus or vaccinia virus, are mutated so as not to block PKR function anymore, the resulting viruses do not infect normal cells due to protein synthesis inhibition by PKR, but they replicate in ras-activated neoplastic cells which lack PKR activities.

Accordingly, the present invention provides a method to remove ras-activated neoplastic cells from a mixed cellular composition by using adenovirus or vaccinia virus as defined in claim 1. The modified or mutated virus selectively replicate in ras-activated neoplastic cells while normal cells are resistant. The adenovirus is mutated in the VA1 region, and the vaccinia virus is mutated in the K3L and/or E3L region.

The viruses can be modified or mutated according to the known structure-function relationship of the viral PKR inhibitors. For example, since the amino terminal region of E3 protein interacts with the carboxy-terminal region domain of PKR, deletion or point mutation of this domain prevents anti-PKR function (Chang et al., 1992, 1993, 1995; Sharp et al., 1998; Romano et al., 1998). The K3L gene of vaccinia virus encodes pK3, a pseudosubstrate of PKR. There is a loss-of-function mutation within K3L. By either truncating or by placing point mutations within the C-terminal portion of K3L protein, homologous to residues 79 to 83 in eIF-2α abolish PKR inhibitory activity (Kawagishi-Kobayashi et al., 1997).

### 3. Removal of viruses after virus treatment

Although the virus used in the present invention does not replicate in normal cells, it may be desired to remove the virus prior to using the virus treated cellular composition.

Accordingly, in another embodiment of this invention, the cellular compositions which have been treated with a virus are frozen in a solution containing DMSO and thawed prior to transplantation. While DMSO is routinely used to freeze and store animal cells, it denatures viruses, thereby removing infectious virus from the stem cell preparation. This reduces the risk that the virus may cause undesired infections when it is introduced into the transplant recipient via stem cell transplantation.

In another embodiment, the virus-treated cell compositions are treated with specific antibodies against the particular virus or a combination of the specific antibodies and complements in order to inactivate or lyse the virus. Alternatively or additionally, specific antibodies which recognize a molecule on the surface of the particular virus may be used to remove the virus particles from the virus-treated cellular composition. Thus, the antibodies are immobilized to a column, beads or any other material or device known in the art, the cellular composition is applied to the immobilzed antibodies, and the part of the composition which does not bind to the antibodies is collected according to a procedure suitable for the particular method of immobilization.

Another method which may be used to remove the virus from virus-treated mixture is to subject the mixture to a gradient which separates cells from the virus, and collect the layer that contains only the cells.

In another embodiment, the transplant recipient is given treatments to stimulate the immune system in order to reduce the risk of virus infection. This treatment may be performed prior to, contemporaneously with, or after the transplantation, but is preferably performed prior to the transplantation. As an alternative treatment or in conjunction with the immune system stimulant, the recipient can be given specific antibodies against the particular virus in order to reduce the risk of virus infection.

### Composition

Also described herein is a composition which is prepared by subjecting a mixed cellular composition to virus treatment wherein the virus results in substantial killing of the neoplastic cells contained in this cellular composition. This composition is not a viral oncolysate. A viral oncolysate is the composition resulting from oncolysis of tumor cells by a virus, containing as the active component virus-modified tumor cell membranes. In the present invention, by contrast, the active components in a virus-treated cellular composition are the surviving non-neoplastic cells.

### Kit

All the viruses discussed above can be used to purge mixed cellular compositions which may contain neoplastic cells. If desired, it may be determined first which virus or viruses can be used to purge the particular cellular composition. For example, when the mixed cellular composition comprises hematopoietic stem cells obtained from a cancer patient, a biopsy of the cancer can be harvested in advance and tested with different viruses to determine which virus can efficiently kill the cancer cells. The virus can then be used to purge the hematopoietic stem cells.

Alternatively, the mixed cellular composition may be treated with a cocktail of viruses without first determining the efficacy of each virus.

The following examples are offered to illustrate this invention and are not to be construed in any way as limiting the scope of the present invention.

### EXAMPLES

In the examples below, the following abbreviations have the following meanings. Abbreviations not defined have their generally accepted meanings.
- °C =: degree Celsius
- hr =: hour
- min =: minute
- µM =: micromolar
- mM =: millimolar
- M =: molar
- ml =: milliliter
- µl =: microliter
- mg =: milligram
- µg =: microgram
- PAGE =: polyacrylamide gel electrophoresis
- rpm =: revolutions per minute
- FBS =: fetal bovine serum
- DTT =: dithiothrietol
- SDS =: sodium dodecyl sulfate
- PBS =: phosphate buffered saline
- DMEM =: Dulbecco's modified Eagle's medium
- α-MEM =: α-modified Eagle's medium
- β-ME =: β-mercaptoethanol
- MOI =: multiplicity of infection
- PFU =: plaque forming units
- PKR =: double-stranded RNA activated protein kinase
- EGF =: epidermal growth factor
- PDGF =: platelet derived growth factor
- DMSO=: dimethylsulfoxide
- CPE =: cytopathic effect
- GCSF =: granulocyte colony stimulating factor

### Example 1 Reference Reovirus induced oncolysis and apoptosis in breast cancer cells

To determine the effect of reovirus on the viability of neoplastic cells, we first used three breast cancer model systems, MCF7 (ATCC number HTB-22), SKBR3 (ATCC number HTB-30) and MDA MB 468 (ATCC number HTB 132). Cells of each cell line were grown to 50-60% confluency and infected with reovirus serotype 3, strain Dearing, at a multiplicity of infection of 40. Reovirus was obtained and maintained as described in U.S. Patent No. 6,136,307. Reovirus infected and non-infected cells were harvested at 0, 24, 48 and 72 hours after infection and the viability was determined.

The results are shown in Figures 1A-1D. Viable cell count in reovirus-infected MCF7 (Figure 1A). SKBR3 (Figure 1B) or MDA MB 468 cells (Figure 1 C) dropped significantly after the infection, while the cells infected with dead virus or no virus proliferated as expected. Reovirus treatment caused MCF7 (Figure 1D) and SKBR3 viability to drop from 93% to 16% by 72 hours after infection. In MDA MB 468 cells, virus treated intact cell numbers dropped to 12.7%, 8.8% and 3.6% of the original cell counts, respectively, at 24, 48 and 72 hours after infection. Thus, reovirus caused oncolysis efficiently in all-three kinds of cancer cells.

The cells died by apoptosis. Typical apoptotic markers such as CPE, Annexin-V and DNA laddering could be observed in a time course parallel to the decrease of viability. Figures 2A-2G show the percentage of DNA fragmentation (2A-2C), Annexin V staining (2D) or AP02.7⁺ cells (2E-2G) at various time points after reovirus infection. The reovirus treated cells exhibited all signs of apoptosis at a dramatic level compared to the no virus or dead virus controls, demonstrating that reovirus induced apoptosis in all of these three cell lines. Apoptosis in the controls seemed to increase slowly with time as well, probably because cells began to die when they had grown too densely.

### Example 2 (Reference) Reovirus selectively inhibited protein synthesis in cancer cells but not CD34⁺ stem cells

For further proof of selective viral infection of cancer cells, ³⁵S labeling/SDS/PAGE of viral proteins was undertaken. Viral protein synthesis was evident after 1-2 days in MCF7 cells infected with reovirus, while cellular protein synthesis decreased at the same time, indicating that reovirus had taken over the cellular machinery. At 4 days after infection, no protein synthesis could be detected anymore, suggesting that all the cells had been killed. In the-control experiments where cells were infected with dead reovirus or no virus, there was no viral protein synthesis, whereas cellular protein synthesis was at the normal level. In contrast, ³⁵S labeling of CD34⁺ stem cells in the presence or absence of reovirus showed no viral protein synthesis up to 72 hours after the addition of virus. Therefore, reovirus selectively infect MCF7 cells but not CD34⁺ stem cells.

### Example 3 (Reference) Reovirus treatment neither inhibited cell proliferation nor altered differentiation potential of CD34⁺ cells

Consistent with the protein synthesis results, viable cell count indicated that reovirus treatment did not decrease the number of viable cells in CD34⁺ cells (Figure 3A) as compared to the no virus control.

While the number of CD34⁺ cells was unaffected by reovirus infection, there remained the question whether reovirus changed the potential of CD34⁺ stem cells to differentiate into all the hematopoietic lineages in the appropriate proportion. If this was the case, reovirus treated stem cells would not be a good candidate for the reconstitution of the whole hematopoietic system. To investigate this possibility, CD34⁺ cells were incubated with reovirus for 2, 24, 48 or 72 hours, respectively. The reovirus was then removed and the cells were diluted and cultured in fresh media for 14 days to allow colonies to form. Each colony was examined to determine if it belongs to the granulocyte, erythroid, or granulocyte erythroid macrophage megakaryocyte lineage. As shown in Figure 3B, stem cells treated with live virus (LV) yielded similar numbers of granulocutes (G), erythrocytes (E) or granulocyte erythroid macrophage megakaryocytes (GEM) as the no virus (NV) control. Therefore, reovirus treatment did not change the differentiation potential of CD34⁺ cells.

### Example 4 (Reference) Reovirus selectively removed cancer cells from a mixed cellular composition

Neoplastic cells were mixed with apheresis product and subjected to reovirus infection to investigate if reovirus can selectively remove neoplastic cells from the mixed cellular composition. Apheresis product was prepared according to a procedure previously described (Stewart et al., 1999; Duggan et al., 2000). When admixtures of apheresis product (90%) and MCF7 (10%) were treated with reovirus and tested daily for cell count and viability, there was a 100-fold depletion in the numbers of cytokeratin-positive MCF7 cells while the CD34⁺ stem cells remained intact and viable. Figures 4A-4C show the purging effect of reovirus to mixtures of apheresis product with MCF7, SKBR3 or MDA MB 468 cells. These results demonstrate that reovirus can selectively kill neoplastic cells in a cell mixture and leave the stem cells intact.

## Claims

1. A method of selectively removing ras-activated neoplastic cells from a mixed cellular composition wherein said composition is located outside of a living organism, said method comprising the steps of:
(a) contacting the mixed cellular composition comprising the ras-activated neoplastic cells with a virus selected from the group consisting of adenovirus mutated in the VAI gene and vaccinia virus mutated in the K3L gene and/or E3L gene under conditions which result in substantial killing of the ras-activated neoplastic cells so as to selectively remove the ras-activated neoplastic cells from the compostion; and
(b) collecting the treated composition.

2. The method of Claim 1 wherein the mixed cellular composition comprises hematopoietic stem cells.

3. The method of Claim 2 wherein the hematopoietic stem cells have been harvested from bone marrow.

4. The method of Claim 2 wherein the hematopoietic stem cells have been harvested from blood.

5. The method of Claim 1 wherein the cellular composition comprises a tissue, an organ or any portion of a tissue or an organ.

6. The method of Claim 5 wherein the tissue or organ is selected from the group consisting of liver, kidney, heart, cornea, skin, lung, pancreatic islet cells, and whole blood.

7. The method of Claim 5 wherein the tissue, organ or portion of the rissue or organ is useful for transplantation.

8. The method of Claim 1 wherein the cellular composition comprises cultured cells, semen or eggs.

9. The method of Claim 1 wherein the virus is a replication competent virus.

10. The method of Claim 1 wherein the virus is mutated or modified such that the virus does not produce a gene product which inhibits double stranded RNA kinase (PKR).

11. The method of Claim 1 further comprising the step of removing the virus from the virus treated cellular composition.

12. The method of Claim 1 further comprising the step of storing the virus treated cellular composition.

13. The method of Claim 12 wherein the cellular composition is stored in a solution containing DMSO.

## Patentansprüche

1. Verfahren zum selektiven Entfernen von ras-aktivierten neoplastischen Zellen aus einer gemischten zellulären Zusammensetzung, **dadurch gekennzeichnet, dass** sich die Zusammensetzung außerhalb eines lebenden Organismus befindet, wobei das Verfahren folgende Schritte umfasst:
(a) Kontaktieren der gemischten zellulären Zusammensetzung, die die ras-aktivierten neoplastischen Zellen umfasst, mit einem Virus ausgewählt aus der Gruppe bestehend aus Adenvirus mit Mutation im VAI-Gen, Vaccina-Virus mit Mutation im K3L-Gen und/oder im E3L-Gen, unter Bedingungen, die zum Abtöten der ras-aktivierten neoplastischen Zellen führen, um so selektiv die ras-aktivierten neoplastischen Zellen aus der Zusammensetzung zu entfernen, und
(b) Gewinnen der behandelten Zusammensetzung.

2. Verfahren nach Anspruch 1, wobei die gemischte zelluläre Zusammensetzung hämatopoetische Stammzellen umfasst.

3. Verfahren nach Anspruch 2, wobei die hämatopoetischen Stammzellen aus Knochenmark geerntet wurden.

4. Verfahren nach Anspruch 2, wobei die hämatopoetischen Zellen aus Blut geerntet wurden.

5. Verfahren nach Anspruch 1, wobei die zelluläre Zusammensetzung ein Gewebe, ein Organ oder einen Teil eines Gewebes oder Organs umfasst.

6. Verfahren nach Anspruch 5, wobei das Gewebe oder das Organ ausgewählt ist aus der Gruppe bestehend aus Leber, Niere, Herz, Kornea, Haut, Lunge, pankreatischen Inselzellen und Vollblut.

7. Verfahren nach Anspruch 5, wobei das Gewebe, Organ oder der Teil des Gewebes oder des Organs nützlich bei Transplantationen ist.

8. Verfahren nach Anspruch 1, wobei die zelluläre Zusammensetzung kultivierte Zellen, Samen oder Eier umfasst.

9. Verfahren nach Anspruch 1, wobei das Virus ein replikationskompetentes Virus ist.

10. Verfahren nach Anspruch 1, wobei das Virus Mutationen oder Modifikationen aufweist, so dass das Virus kein Genprodukt produziert, das doppelsträngige RNA-Kinase (PKR) inhibiert.

11. Verfahren nach Anspruch 1, weiter umfassend den Schritt des Entfernens des Virus von der mit dem Virus behandelten Zusammensetzung.

12. Verfahren nach Anspruch 1, weiter umfassend den Schritt des Lagerns der mit dem Virus behandelten Zusammensetzung.

13. Verfahren nach Anspruch 12, wobei die zelluläre Zusammensetzung in einer DMSO-haltigen Lösung gelagert wird.

## Revendications

1. Procédé d'élimination sélective de cellules néoplasiques activées par ras d'une composition cellulaire mélangée, ladite composition étant située à l'extérieur d'un organisme vivant, ledit procédé comprenant les étapes consistant à :
(a) mettre ladite composition cellulaire mélangée contenant les cellules néoplasiques activées par ras en contact avec un virus choisi dans l'ensemble consistant en adénovirus portant une mutation dans le gène VAI et virus de la vaccine portant une mutation dans le gène K3L et/ou le gène E3L dans des conditions qui permettent une destruction substantielle des cellules néoplasiques activées par ras de façon à éliminer sélectivement de la composition les cellules néoplasiques activées par ras ; et
(b) la collecte de la composition traitée.

2. Procédé de la revendication 1, dans lequel la composition cellulaire mélangée comprend des cellules souches hématopoïétiques.

3. Procédé de la revendication 2, dans lequel les cellules souches hématopoïétiques ont été récoltées à partir de moelle osseuse.

4. Procédé de la revendication 2, dans lequel les cellules souches hématopoïétiques ont été récoltées à partir de sang.

5. Procédé de la revendication 1, dans lequel la composition cellulaire comprend un tissu, un organe ou une partie quelconque d'un tissu ou d'un organe.

6. Procédé de la revendication 5, dans lequel le tissu ou l'organe est choisi dans l'ensemble constitué du foie, du rein, du coeur, de la cornée, de la peau, du poumon, d'îlots de Langerhans pancréatiques, et de sang entier.

7. Procédé de la revendication 5, dans lequel le tissu, l'organe ou la partie de tissu ou d'organe est utile pour la greffe.

8. Procédé de la revendication 1, dans lequel la composition cellulaire comprend des cellules en culture, du sperme ou des ovules.

9. Procédé de la revendication 1, dans lequel le virus est un virus compétent pour la réplication.

10. Procédé de la revendication 1, dans lequel le virus est muté ou modifié de sorte que le virus ne produise pas de produit génique qui inhibe la protéine kinase dépendante de l'ARN bicaténaire (PKR).

11. Procédé de la revendication 1, qui comprend en outre l'étape d'élimination du virus de la composition cellulaire traitée par le virus.

12. Procédé de la revendication 1, qui comprend en outre l'étape de stockage de la composition cellulaire traitée par le virus.

13. Procédé de la revendication 12, dans lequel la composition cellulaire est stockée dans une solution contenant du DMSO.
